# EUROPEAN PATENT SPECIFICATION

(11) **EP 3 459 530 B1**
(45) Date of publication and mention of the grant of the patent: **23.09.2020**
(21) Application number: 18199791.7
(22) Date of filing: 13.05.2010
(51) Int. Cl.: A61K 9/20, A61K 31/192

(54) **BURST DRUG RELEASE COMPOSITIONS**
DURCH BERSTEN FREIGESETZTE ARZNEIMITTELZUSAMMENSETZUNGEN
COMPOSITIONS À LIBÉRATION DE MÉDICAMENT EN RAFALE

(30) Priority: 13.05.2009 US 17794309 P
(43) Date of publication of application: 27.03.2019
(62) Divisional of application: 10720087.5
(73) Proprietor: PF Consumer Healthcare 1 LLC, Wilmington, DE 19801 (US)
(72) Inventor: KINTER, Kevin, Glen Allen, VA Virginia 23059 (US); RAMSEY, Peter, Moselev, VA Virginia 23120 (US)
(74) Representative: Morris, Miriam Elizabeth

(56) References cited:
- WO-A2-2008/069938
- US-A- 4 871 548
- VELASCO M V ET AL: "Effect of media on the dissolution profiles of propranolol hydrochloride from matrices containing different substitution types of Methocel", PHARMACY AND PHARMACOLOGY COMMUNICATIONS 1998 GB, vol. 4, no. 8, 1998, pages 377-383, XP009138268, ISSN: 1460-8081
- REITZ C ET AL: "Solid lipid extrudates as sustained-release matrices: The effect of surface structure on drug release properties", EUROPEAN JOURNAL OF PHARMACEUTICAL SCIENCES, ELSEVIER, AMSTERDAM, NL LNKD- DOI:10.1016/J.EJPS.2008.08.002, vol. 35, no. 4, 15 November 2008 (2008-11-15), pages 335-343, XP025560081, ISSN: 0928-0987 [retrieved on 2008-08-14]

## Description

### BACKGROUND

Burst drug release from extended release hydrophilic matrix tablets is an evolving area of pharmaceutics. The pharmaceutical industry employs various methods for compounding pharmaceutical agents in tablet formulations. In addition to active ingredients, formulations include other excipients such as controlled release agents, diluents, binders, disintegrants, surface active agents, glidants, lubricants, colorants, coating substances, surfactants and many other raw materials that impart different properties to the final solid dosage product.

Further, certain processing steps are utilized to formulate and accurately formulate and/or manufacture solid dose products. The most common processing steps associated with preparing solid dose formulations are summarized below:
"Wet Granulation" methods can be used where the flow properties of a compound such as an active pharmaceutical ingredient ("API") are poor which result in content uniformity issues when formulated as a dry blend. Wet granulation is commonly used to improve the processing characteristics of a powder blend, including improved flowability, content uniformity and more uniform particle size.

Wet granulation is used to improve flow, compressibility, bio-availability, homogeneity, electrostatic properties, and stability of solid dosage forms. Granulation is often required to improve the flow of powder mixtures and mechanical properties of tablets. Granules are usually obtained by adding liquids (binder or solvent solutions). Larger quantities of granulating liquid produce a narrower particle size range and coarser and harder granules, i.e. the proportion of fine granulate particles decreases. The particle size of the granulate is determined by the quantity and feeding rate of granulating liquid.

Wet granulation methods can be used where the flow properties of a compound such as an active pharmaceutical ingredient ("API") are poor which result in content uniformity issues when formulated as a dry blend. Wet granulation is commonly used to improve the processing characteristics of a powder blend, including improved flowability, content uniformity and more uniform particle size. The use of water and heat in wet granulation may cause chemical degradation or physical form conversion.

The variables faced in the processing of the granules can lead to significant tableting problems. Properties of granules formed can be affected by viscosity of granulating solution, the rate of addition of granulating solution, type of mixer used and duration of mixing, method and rate of dry and wet blending. The above variables can change the density and the particle size of the resulting granules and may have a major influence on fill weight and compaction qualities. Drying can lead to an unfavorable separation as soluble API migrates to the surface of the drying granules.

"Direct Compression" is defined as the process by which tablets are compressed directly from powder mixture of API and suitable excipients. No pretreatment of the powder blend by wet or dry granulation procedure is required. It involves only blending and compression. This offers the advantage of speedy production because it requires fewer unit operations, less machinery, and generally less processing time along with, in some cases, increased product stability.

In case of directly compressed tablets after disintegration, each primary drug particle is liberated. While in the case of tablets prepared by compression of granules, small drug particles with a larger surface area adhere together into larger agglomerates; thus decreasing the surface area available for dissolution.

While having all the benefits a granulation process can provide such as improving material flow behavior and content uniformity, "Roller Compaction" offers unique advantages over wet granulation for moisture, solvent or heat sensitive compounds.

In roller compaction, powder is fed to two counter-rotating rolls which draw the powder between the rolls due to friction, which compacts the powder. Roller compaction is seemingly a simple process but the fundamental mechanisms are complex due to a number of material properties and machine variables involved such as material flow properties, friction against roll surface, compressibility, compactibility, elastic properties, air permeability, roll surface, roll dimension, roll pressure, roll gap, roll speed, feed method and conditions.

There are generally three controllable parameters in the roller compaction process: roll pressure, roll gap and roll speed. Because the consolidation of a powder blend into ribbons is the result of mechanical stress (normal and shear stresses) within the powder during roller compaction, all the parameters are studied by examining their correlation to the normal (compressive) stress and the shear stress.

Viscosity is another characteristic that is relevant to solid dosage pharmaceutical compositions, though viscosity is most commonly recognized as property which characterizes the flow nature of a liquid. In the pharmaceutical arts, viscosity becomes relevant with respect to solid dosage forms such as tablets and capsules once these are taken orally and are exposed to the fluids in the digestive tract including the mouth, throat, stomach and intestines.

Controlled release agents are commonly included as excipients in pharmaceutical formulations. Such sustained release agents, preferably a substituted cellulose derivative, such as hydroxypropylmethyl cellulose (HPMC) facilitate the delayed release of the pharmaceutically active ingredients from the formulation such that the formulation can be administered to a patient less often, such as once daily. It is preferably present in an amount that allows for the formation of a gel matrix from which the active ingredient is gradually released. In addition, composition contemplated herein may comprise further sustained release agents, preferably those that swell upon contact with water such as polyvinylpyrrolidone, hydroxyethylcellulose, hydroxypropylcellulose, other cellulose ethers and esters like methylcellulose, methylethylcellulose, hydroxypropylmethylcellulose, carboxymethylcellulose, starch, pregelatinized starch, polymethacrylate, polyvinylacetate, microcrystalline cellulose, dextrans or mixtures thereof.

### SUMMARY OF THE INVENTION

The inventors have found that certain kinds of processing on ingredients in formulations that included pharmaceutical actives and controlled release agents with certain apparent viscosities have marked effect on the dissolution of the active ingredients. In the pharmaceutical sciences, controlling the dissolution rate of active ingredients can be critical to the desired release timing and functionality of the active ingredient(s). As such, the discoveries and compositions disclosed herein offer novel approaches to controlling the dissolution of active ingredients through unique combinations of ingredients and using specifically processed active ingredient(s) in the compositions.

The invention provides solid dose compositions as defined in claim 1.

### DESCRIPTION OF THE INVENTION AND EXAMPLES

One specific observation and advantage of the inventions disclosed herein is that the process of wet granulation of the mix of pharmaceutically active ingredients plus controlled release agents gave surprising results in solid formulations with respect to the burst characteristics of pharmaceutically active ingredients at certain viscosities. Specifically, higher viscosity formulations where the mix of pharmaceutically active ingredients plus controlled release agents were processed using wet granulation had consistently faster burst rates.

Conversely, another specific observation and advantage of the inventions disclosed herein is that the processes of direct compression and roller compaction showed similar characteristics to each other with respect to burst characteristics of pharmaceutically active ingredients at certain viscosities. Specifically, higher viscosity formulations where the mix of pharmaceutically active ingredients plus controlled release agents were processed using direct compression and roller compaction had consistently slower burst rates than those processed using wet granulation.

The invention will allow the formulation of pharmaceuticals wherein release profiles can be adjusted to create compositions with both Immediate Release (IR) and Extended Release (ER) characteristics. As a preferred embodiment, with respect to pain management products, a critical need is to have an initial dose released up front to provide analgesia along with efficacious blood levels for sustained time periods. Compositions, exemplified herein, offer such advantageous characteristics.

A further advantage to the compositions embodied by this invention is that IR/ER formulations can be created in a single, monolithic design. Contrary to commonly used multilayer approaches to IR/ER formulations, monolithic prototypes have significant advantages from a manufacturing perspective. The tablet/capsule press can be run faster as the compression events that are occurring are less complicated. Additionally, with tablet presses that are double sided, production rates can be doubled. Also, tablet specifications are more straight forward as you are only dealing with a single layer. As such, there is less concern with layers sticking to one another as can occur with bilayers. Further, bilayer tablets may have higher friability.

With regard to the at least one active ingredient, the present invention comprises ibuprofen which may be present in the claimed compositions in amounts ranging from about 50 to about 800 mg. Preferably it is present in amounts ranging from about 200 to about 600 mg. Most preferably, it is present in an amount of about 600 mg. The exact amount required will vary from subject to subject, depending on age, general condition of the subject, the severity of the condition being treated, and the particular active agent administered, and the like.

Compositions of the invention are formulated in a solid single dosage form such as tablets, capsules, sachets, trochets and the like. Solid compounds will typically be administered orally.

Exemplary compositions of the present invention are directed to solid dosage forms such as bulk powders, tablets, caplets, pellets, capsules, sachets, granules, and any other dosage form suitable for oral administration. For purposes of this specification and the accompanying claims, the term "tablet" refers equally to a tablet, a caplet or any other solid dosage form which is suitable for oral administration.

The compositions of the present invention also include one or more non-pharmaceutically active excipients. These include, but are not limited to, controlled release agents, diluents, binders, disintegrants, surface active agents, glidants, lubricants, colorants, coating substances, surfactants and many other raw materials that impart different properties to the final solid dosage product.

At least one controlled release agent is included which is the substituted cellulose derivative hydroxypropylmethyl cellulose (HPMC) which facilitates the delayed release of the pharmaceutically active ingredients from the formulation such that the formulation can be administered to a patient less often, such as once daily. It is present in an amount of 20% to 30% by weight of the final composition and has an apparent viscosity between 100 and 100,000 centipoise. In addition, composition contemplated herein may comprise further sustained release agents, preferably those that swell upon contact with water such as polyvinylpyrrolidone, hydroxyethylcellulose, hydroxypropylcellulose, other cellulose ethers and esters like methylcellulose, methylethylcellulose, hydroxypropylmethylcellulose, carboxymethylcellulose, starch, pregelatinized starch, polymethacrylate, polyvinylacetate, microcrystalline cellulose, dextrans or mixtures thereof.

Binders are agents used to impart cohesive qualities to the powdered material. Binders impart cohesiveness to the tablet formulation which insures the tablet remaining intact after compression, as well as improving the free-flowing qualities by the formulation of granules of desired hardness and size. Suitable binder materials include, but are not limited to, starch (including corn starch and pregelatinzed starch), gelatin, sugars (including sucrose, glucose, dextrose, lactose and sorbitol), polyethylene glycol, waxes, natural and synthetic gums, e.g., acacia, tragacanth, sodium alginate, celluloses such as Microcrystalline Cellulose and synthetic polymers such as polymethacrylates and polyvinylpyrrolidone.

Lubricants have a number of functions in tablet manufacture. They prevent adhesion of the tablet material to the surface of the dies and punches, reduce interparticle friction, facilitate the ejection of the tablets from the die cavity and may improve the rate of flow of the tablet granulation. Examples of suitable lubricants include, but are not limited to, magnesium stearate, calcium stearate, stearic acid, glyceryl behenate, talc, sodium lauryl sulfate, sodium stearyl fumarate, polyethylene glycol or mixtures thereof. Generally, the lubricant is present in an amount from about 0.25% to about 5% of the weight of the final composition and more specifically from about 0.5 to about 1.5% of the weight of the final composition.

A disintegrant is a substance, or a mixture of substances, added to a tablet to facilitate its breakup or disintegration after administration. Materials serving as disintegrants have been classified chemically as starches, clay, celluloses, aligns, gums and cross-linked polymers. Examples of suitable disintegrants include, but are not limited to, crosscarmelose sodium, sodium starch glycolate, starch, magnesium aluminum silicate, colloidal silicon dioxide, methylcellulose, agar, bentonite, alginic acid, guar gum, citrus pulp, carboxymethyl cellulose, microcrystalline cellulose, or mixtures thereof. Generally, the disintegrant is present in an amount from 0% to about 30% of the weight of the final composition and more specifically from about 0% to about 15% of the weight of the final composition.

Glidants are substances which improve the flow characteristics of a powder mixture. Examples of glidants include, but are not limited to colloidal silicon dioxide, talc or mixtures thereof. Generally, the glidant is present in an amount of from about 0.1 % to about 10% of the weight of the final composition and more specifically from 5 about 0.1% to about 5% of the weight of the final composition.

The adsorbent may be, for example colloidal silicon dioxide, microcrystalline cellulose, calcium silicate or mixtures thereof. Generally, the adsorbent is present in an amount from about 0.05% to about 42% of the weight of the final composition and more specifically from about 0.05% to about 37% of the weight of the final composition.

If desired, other ingredients, such as diluents, stabilizers and anti-adherents, conventionally used for pharmaceutical formulations may be included in the present formulations. Optional ingredients include coloring and flavoring agents which are well known in the art.

The pharmaceutical composition described in the present invention may be formulated to release the active ingredients in a sustained release manner. Various formulations are contemplated for dosage forms of these components.

The invention is further described by means of the following examples, which are not intended to limit the scope of the claimed invention in any manner.

### EXAMPLES

The following embodiments demonstrate the advantages of the inventions.

To investigate process effects, small scale lab batches of monolithic prototypes were manufactured by wet granulation (WG), roller compaction (RC) comparative examples), and direct compression (DC) (comparative examples). To investigate polymer effects, a matrix of different viscosity grades (K100LV and K4M) and levels of hydroxypropyl methylcellulose (20 and 25% HPMC) was evaluated. See Table 1 for the premix preparations used in the specific Examples below.

**Table 1: Premix preparations used in the examples**

| Premix Batch A: 20% HPMC (100:0 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 67.26 | 600.00 |
| MCC, NF (Avicel pH 102) | 12.56 | 112.00 |
| HPMC, USP K100LV Premium CR | 20.18 | 180.00 |
| HPMC, USP K4M Premium CR | 0.00 | 0.00 |
| TOTAL | 100.00 | 892.00 |

| Premix Batch B: 20% HPMC (67:33 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 67.26 | 600.00 |
| MCC, NF (Avicel pH 102) | 12.56 | 112.00 |
| HPMC, USP K100LV Premium CR | 13.45 | 120.00 |
| HPMC, USP K4M Premium CR | 6.73 | 60.00 |
| TOTAL | 100.00 | 892.00 |

| Premix Batch C: 20% HPMC (33:67 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 67.26 | 600.00 |
| MCC, NF (Avicel pH 102) | 12.56 | 112.00 |
| HPMC, USP K100LV Premium CR | 6.73 | 60.00 |
| HPMC, USP K4M Premium CR | 13.45 | 120.00 |
| TOTAL | 100.00 | 892.00 |

| Premix Batch D: 20% HPMC (0:100 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 67.26 | 600.00 |
| MCC, NF (Avicel pH 102) | 12.56 | 112.00 |
| HPMC, USP K100LV Premium CR | 0.00 | 0.00 |
| HPMC, USP K4M Premium CR | 20.18 | 180 |
| TOTAL | 100.00 | 892.00 |

| Premix Batch E: 25% HPMC (100:0 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 63.06 | 600.00 |
| MCC, NF (Avicel pH 102) | 11.72 | 111.50 |
| HPMC, USP K100LV Premium CR | 25.22 | 240.00 |
| HPMC, USP K4M Premium CR | 0.00 | 0.00 |
| TOTAL | 100.00 | 951.50 |

| Premix Batch F: 25% HPMC (67:33 K100LV:K4M) | | |
|---|---|---|
| | | |
| ∼ Batch Size | 4 | Kilograms |
| ∼ API Dose | 600.00 | mgs |
| Ingredient | w/w % | mg/dose |
| Ibuprofen USP 90 Grade (BASF) | 63.06 | 600.00 |
| MCC, NF (Avicel pH 102) | 11.72 | 111.50 |
| HPMC, USP K100LV Premium CR | 16.82 | 160.00 |
| HPMC, USP K4M Premium CR | 8.41 | 80.00 |
| TOTAL | 100.00 | 951.50 |

| Premix Batch G: 25% HPMC (33:67 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 63.06 | 600.00 |
| MCC, NF (Avicel pH 102) | 11.72 | 111.50 |
| HPMC, USP K100LV Premium CR | 8.41 | 80.00 |
| HPMC, USP K4M Premium CR | 16.82 | 160.00 |
| TOTAL | 100.00 | 951.50 |

| Premix Batch H: 25% HPMC (0:100 K100LV:K4M) | | |
|---|---|---|
| ∼ Batch Size | 4 | Kilograms |
| ∼ API Dose | 600.00 | mgs |
| Ingredient | w/w % | mg/dose |
| Ibuprofen USP 90 Grade (BASF) | 63.06 | 600.00 |
| MCC, NF (Avicel pH 102) | 11.72 | 111.50 |
| HPMC, USP K100LV Premium CR | 0.00 | 0.00 |
| HPMC, USP K4M Premium CR | 25.22 | 240 |
| TOTAL | 100.00 | 951.50 |

The following compositions were then formulated. The premixes A - H (in Table I) were blended and portions of each premix were distributed to the three manufacturing processes. The direct compression premix was blended with silicon dioxide and stearic acid and compressed (as described below). The roller compaction premixes were granulated and milled on lab scale equipment and then blended with the extragranular silicon dioxide and stearic and compressed. The wet granulation premixes were granulated, dried and milled on lab scale equipment and then blended with the extragranular silicon dioxide and stearic and compressed.
Example 1: Direct Compression Batch A (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend A | 98.67 | 892.00 |
| Silicon Dioxide Colloidal NF erosol 200 | 0.88 | 8.00 |
| Stearic Acid, NF Powder Food Grade | 0.44 | 4.00 |
| TOTAL | 100.00 | 904.00 |

Example 2: Direct Compression Batch B (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend B | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 3: Direct Compression Batch C (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend C | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 4: Direct Compression Batch D (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend D | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 5: Direct Compression Batch E (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend E | 98.67 | 951.50 |
| Silicon Dioxide Colloidal NF erosol 200 | 0.88 | 8.53 |
| Stearic Acid, NF Powder Food Grade | 0.44 | 4.27 |
| TOTAL | 100.00 | 964.30 |

Example 6: Direct Compression Batch F (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend F | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Example 7: Direct Compression Batch G (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend G | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Example 8: Direct Compression Batch H (Comparative)

| | | |
|---|---|---|
| ∼ Batch Size | 2 | Kilograms |
| ∼ API Dose | 600.00 | mgs |
| Ingredient | w/w % | mg/dose |
| Ibuprofen Pre-Mix Blend H | 98.67 | 951.50 |
| Silicon Dioxide Colloidal NF erosol 200 | 0.88 | 8.53 |
| Stearic Acid, NF Powder Food Grade | 0.44 | 4.27 |
| TOTAL | 100.00 | 964.30 |

Example 9: Roller Compaction Batch A (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC A | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 10: Roller Compaction Batch B (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC B | 98.67 | 892.00 |
| Silicon Dioxide Colloidal NF erosol 200 | 0.88 | 8.00 |
| Stearic Acid, NF Powder Food Grade | 0.44 | 4.00 |
| TOTAL | 100.00 | 904.00 |

Example 11: Roller Compaction Batch C (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC C | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 12: Roller Compaction Batch D (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC D | 98.67 | 892.00 |
| Silicon Dioxide Colloidal NF erosol 200 | 0.88 | 8.00 |
| Stearic Acid, NF Powder Food Grade | 0.44 | 4.00 |
| TOTAL | 100.00 | 904.00 |

Example 13: Roller Compaction Batch E (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC E | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Example 14: Roller Compaction Batch F (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC F | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Example 15: Roller Compaction Batch G (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC G | 98.67 | 951.50 |
| Silicon Dioxide Colloidal NF erosol 200 | 0.88 | 8.53 |
| Stearic Acid, NF Powder Food Grade | 0.44 | 4.27 |
| TOTAL | 100.00 | 964.30 |

Example 16: Roller Compaction Batch H (Comparative)

| | | |
|---|---|---|
| ∼ Batch Size | 0.9 | Kilograms |
| ∼ API Dose | 600.00 | mgs |
| Ingredient | w/w % | mg/dose |
| Ibuprofen Milled RC H | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Example 17: Wet Granulation Batch A

| | | |
|---|---|---|
| ∼ Batch Size | 1 | Kilograms |
| ∼ API Dose | 600.00 | mgs |
| Ingredient | w/w % | mg/dose |
| Ibuprofen Milled WG A | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 18: Wet Granulation Batch B

| | | |
|---|---|---|
| ∼ Batch Size | 1 | Kilograms |
| ∼ API Dose | 600.00 | mgs |
| Ingredient | w/w % | mg/dose |
| Ibuprofen Milled WG B | 98.67 | 892.00 |
| Silicon Dioxide Colloidal NF erosol 200 | 0.88 | 8.00 |
| Stearic Acid, NF Powder Food Grade | 0.44 | 4.00 |
| TOTAL | 100.00 | 904.00 |

Example 19: Wet Granulation Batch C

| | | |
|---|---|---|
| ∼ Batch Size | 1 | Kilograms |
| ∼ API Dose | 600.00 | mgs |
| Ingredient | w/w % | mg/dose |
| Ibuprofen Milled WG C | 98.67 | 892.00 |
| Silicon Dioxide Colloidal NF Aerosil 200 | 0.88 | 8.00 |
| Stearic Acid, NF Powder Food Grade | 0.44 | 4.00 |
| TOTAL | 100.00 | 904.00 |

Example 20: Wet Granulation Batch D

| | | |
|---|---|---|
| ∼ Batch Size | 1 | Kilograms |
| ∼ API Dose | 600.00 | mgs |
| Ingredient | w/w % | mg/dose |
| Ibuprofen Milled WG D | 98.67 | 892.00 |
| Silicon Dioxide Colloidal NF erosol 200 | 0.88 | 8.00 |
| Stearic Acid, NF Powder Food Grade | 0.44 | 4.00 |
| TOTAL | 100.00 | 904.00 |

Example 21: Wet Granulation Batch E

| | | |
|---|---|---|
| ∼ Batch Size | 1 | Kilograms |
| ∼ API Dose | 600.00 | mgs |
| Ingredient | w/w % | mg/dose |
| Ibuprofen Milled WG E | 98.67 | 951.50 |
| Silicon Dioxide Colloidal NF erosol 200 | 0.88 | 8.53 |
| Stearic Acid, NF Powder Food Grade | 0.44 | 4.27 |
| TOTAL | 100.00 | 964.30 |

Example 22: Wet Granulation Batch F

| | | |
|---|---|---|
| ∼ Batch Size | 1 | Kilograms |
| ∼ API Dose | 600.00 | mgs |
| Ingredient | w/w % | mg/dose |
| Ibuprofen Milled WG F | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Example 23: Wet Granulation Batch G

| | | |
|---|---|---|
| ∼ Batch Size | 1 | Kilograms |
| ∼ API Dose | 600.00 | mgs |
| Ingredient | w/w % | mg/dose |
| Ibuprofen Milled WG G | 98.67 | 951.50 |
| Silicon Dioxide Colloidal NF erosol 200 | 0.88 | 8.53 |
| Stearic Acid, NF Powder Food Grade | 0.44 | 4.27 |
| TOTAL | 100.00 | 964.30 |

Example 24: Wet Granulation Batch H

| | | |
|---|---|---|
| ∼ Batch Size | 1 | Kilograms |
| ∼ API Dose | 600.00 | mgs |
| Ingredient | w/w % | mg/dose |
| Ibuprofen Milled WG H | 98.67 | 951.50 |
| Silicon Dioxide Colloidal NF Aerosil 200 | 0.88 | 8.53 |
| Stearic Acid, NF Powder Food Grade | 0.44 | 4.27 |
| TOTAL | 100.00 | 964.30 |

Viscosity* of the specific Examples was determined and summarized in Table 2 below. *Viscosity of a 2% w/w HPMC solution in water, cps

**Table 2: Viscosity Results for HPMC Examples**

| Examples | K100LV | wt % | K4M | Wt % | 2% w/w Soln Visc |
|---|---|---|---|---|---|
| 1, 5, 9, 13, 17, 21 | 100 | 1 | 4000 | 0 | 100 |
| 2, 6, 10, 14, 18, 22 | 100 | 0.67 | 4000 | 0.33 | 411 |
| 3, 7, 11, 15, 19, 23 | 100 | 0.33 | 4000 | 0.67 | 1414 |
| 4, 8, 12, 16,20,24 | 100 | 0 | 4000 | 1 | 4000 |

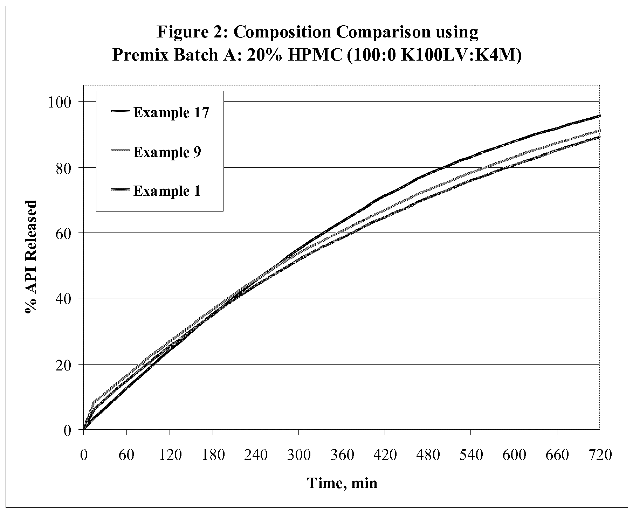

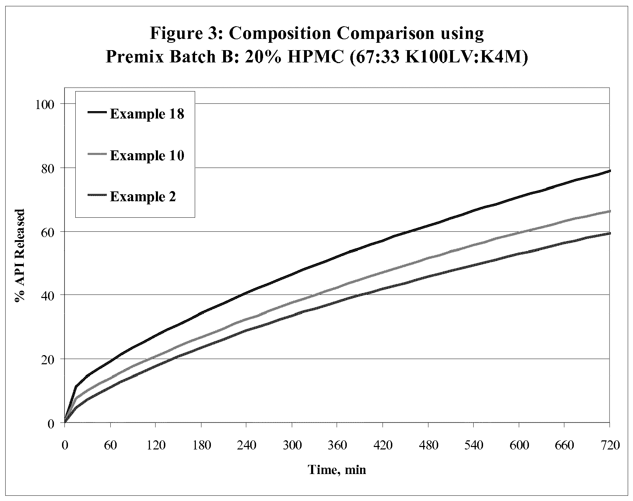

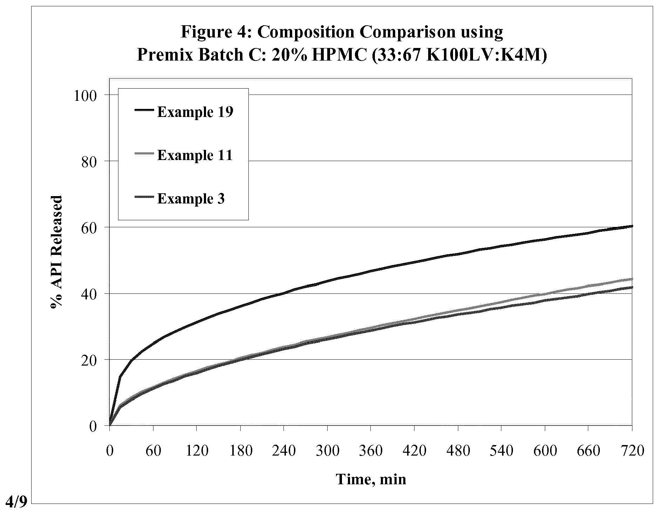

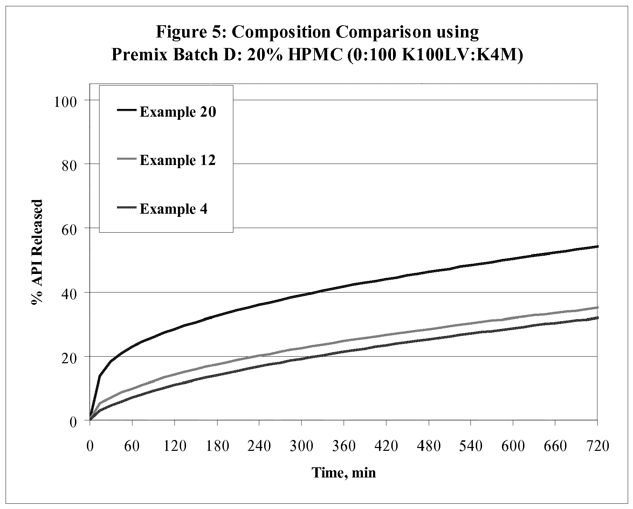

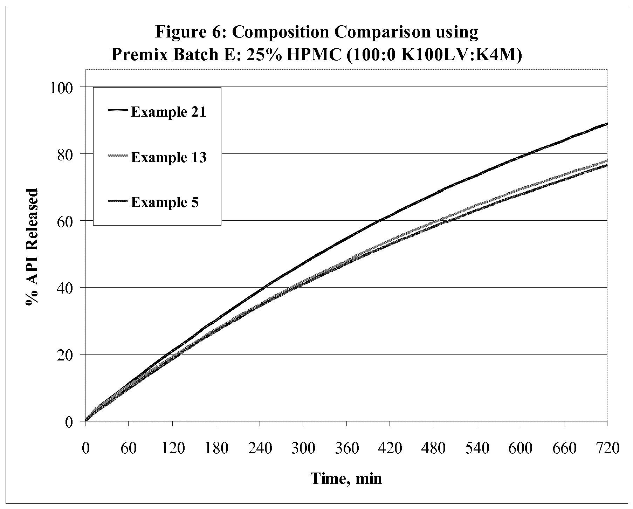

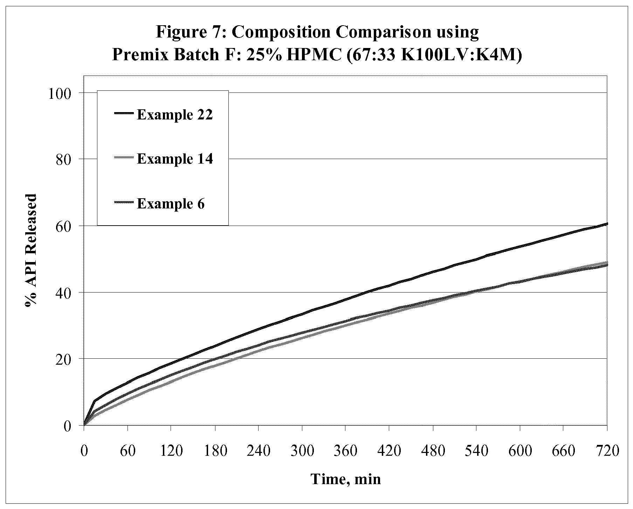

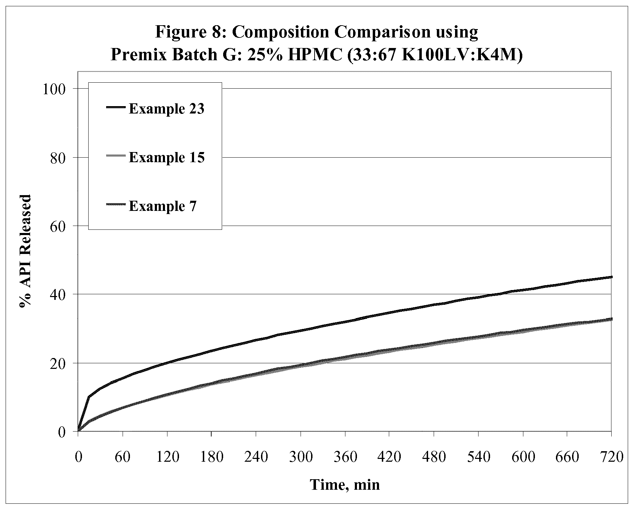

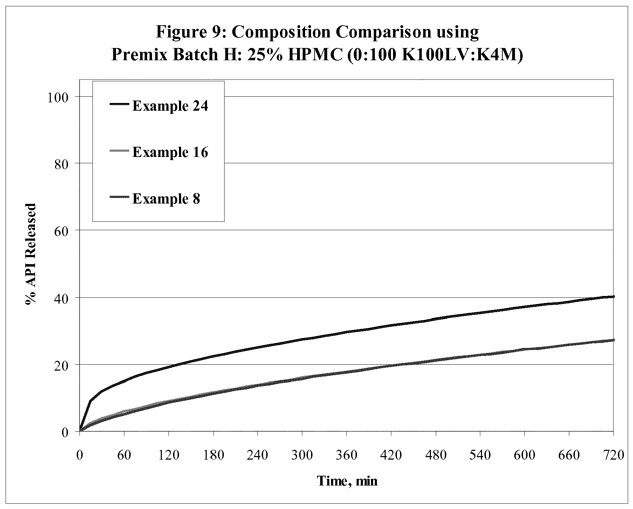

The amount of burst release was expressed as a ratio as shown below, where the denominator is calculated from the linear release rate in the region from 60 to 720 mins. Figures 2-9 show the profiles and comparisons of wet granulation, roller compaction and direct compression processed compositions.

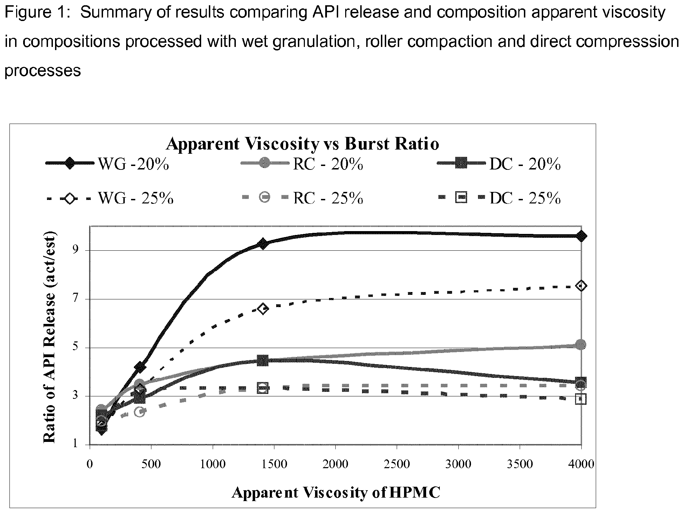

Figure 1 summarizes the comparison API Release and composition viscosity in compositions processed with wet granulation, roller compaction and direct compression processes.

Results: Higher polymer levels were associated with lower release rates and lower levels of burst drug release. As the polymer level increases, the API release rate and burst rate decreases as this creates a more robust gel matrix.

Polymer viscosity also has a strong correlation with burst levels. Increasing the proportion of higher viscosity polymer increases the amount of burst release, which is a function the hydration rates of the HPMC. This reduced hydration level of the polymer allows release of API before the gel matrix develops.

Process factors had the most dramatic effect on burst release. Dry processes had lower levels of burst release vs. wet granulation. These results suggest that the hydration/dehydration steps of the wet granulation process increase the amount of burst release.

Conclusions: This set of BCS Class II, high drug load prototypes showed that burst release can be minimized by using higher levels of HPMC, selecting lower viscosity polymer grades, and using dry processing methods.

## Claims

1. A solid dose composition comprising at least one pharmaceutically active ingredient which is ibuprofen and at least one controlled release agent which is hydroxypropylmethylcellulose and which is present in an amount of 20% to 30% by weight of the final composition wherein the apparent viscosity of the controlled release agent is between 100 and 100,000 centipoise and wherein at least one pharmaceutically active ingredient is processed by wet granulation.

2. A solid dose composition according to claim 1 wherein the apparent viscosity of the controlled release agent is 100 centipoise.

## Patentansprüche

1. Feste Dosierungszusammensetzung, umfassend mindestens einen pharmazeutischen Wirkstoff, der Ibuprofen ist, und mindestens ein Mittel zur kontrollierten Freisetzung, das Hydroxypropylmethylcellulose ist und das in einer Menge von 20 bis 30 Gew.-% der Endzusammensetzung vorhanden ist, wobei die scheinbare Viskosität des Mittels zur kontrollierten Freisetzung zwischen 100 und 100.000 Centipoise beträgt und wobei mindestens ein pharmazeutischer Wirkstoff durch Feuchtgranulierung verarbeitet ist.

2. Feste Dosierungszusammensetzung gemäß Anspruch 1, wobei die scheinbare Viskosität des Mittels zur kontrollierten Freisetzung 100 Centipoise beträgt.

## Revendications

1. Composition de dose solide comprenant au moins un ingrédient pharmaceutiquement actif qui est l'ibuprofène et au moins un agent de libération contrôlée qui est l'hydroxypropylméthylcellulose et qui est présent en une quantité de 20 % à 30 % en poids de la composition finale dans laquelle la viscosité apparente de l'agent de libération contrôlée est entre 100 et 100 000 centipoises et dans laquelle au moins un ingrédient pharmaceutiquement actif est transformé par granulation par voie humide.

2. Composition de dose solide selon la revendication 1 dans laquelle la viscosité apparente de l'agent de libération contrôlée est de 100 centipoises.
